Europäisches Patentamt

European Patent Office

Office européen des brevets

(19)

(11)     EP 0 686 618 B1

(12)                    **EUROPÄISCHE PATENTSCHRIFT**

(45) Veröffentlichungstag und Bekanntmachung des
     Hinweises auf die Patenterteilung:
     **14.01.1998  Patentblatt 1998/03**

(51) Int. Cl.⁶: **C07C 46/04**, C07C 37/60,
     B01J 23/36

(21) Anmeldenummer: 95108050.6

(22) Anmeldetag: 26.05.1995

(54) **Verfahren zur selektiven Oxidation aromatischer Verbindungen**

Process for the selective oxidation of aromatic compounds

Procédé d'oxydation sélective de composés aromatiques

(84) Benannte Vertragsstaaten:
     **CH DE FR GB LI**

(30) Priorität: **06.06.1994 DE 4419800**

(43) Veröffentlichungstag der Anmeldung:
     **13.12.1995  Patentblatt 1995/50**

(73) Patentinhaber:
     **HOECHST AKTIENGESELLSCHAFT
     65929 Frankfurt am Main (DE)**

(72) Erfinder:
     • **Herrmann, Wolfgang Anton, Prof. Dr.
       D-85354 Freising (DE)**
     • **Galamba Correia, Joao Domingos
       D-80796 München (DE)**
     • **Fischer, Richard Walter, Dr.
       D-65929 Frankfurt (DE)**

(56) Entgegenhaltungen:
     **EP-A- 0 508 385**

## Beschreibung

Die Oxidation ist der wichtigste Reaktionstyp in der organischen Synthese. Zahlreiche Grund- und Feinchemikalien resultieren aus oxidativen Prozessen, die sich häufig des Luftsauerstoffs, des Wasserstoffperoxids und der organischen Peroxide als Oxidationsmittel bedienen. In vielen Fällen treten effiziente, selektive Reaktionen nur dann ein, wenn diese Oxidationsmittel in Gegenwart von Katalysatoren verwendet werden. Diese Katalysatoren sind zumeist Metalloxide beispielsweise $V_2O_5$, $CrO_3$, $MoO_3$, $WO_3$, $OsO_4$ und $RuO_4$. Gelegentlich findet man als Oxidationsmittel auch Oxometallate wie Chromat und Permanganat. Die genannten Katalysatoren zeigen große Effizienz in Epoxidations-, Hydroxylierungs- bzw. Carboxylierungsreaktionen an Olefinen (H.A. Jørgensen, Chem. Rev. 1989, Bd. 89, S. 431-458).

Der Einsatz solcher Systeme zur katalytischen Oxidation von aromatischen Verbindungen ist allerdings vielen Beschränkungen unterworfen. Fehlende Aktivität ($WO_3$) einerseits und mangelhafte Selektivität andererseits ($CrO_3/H_2SO_4$) neben der oft nicht gewährleisteten ökologischen sowie gesundheitlichen bzw. pharmakologischen Unbedenklichkeit (z.B. bei $CrO_3$ oder $OsO_4$) verhinderten bislang den technischen Einsatz solcher Katalysatoren.

Andere in der Oxidationschemie etablierte Verfahren, die z.B. mit elektrochemischer Oxidation, Cer(IV)-Salzen, Mangan(III)-sulfat oder Persäuren bzw. Peroxiden (t-BuOOH) in Gegenwart von Molyodänkomplexen als Oxidationsmittel arbeiten, erweisen sich bei der Oxidation von einfachen oder kondensierten Aromaten bzw. deren Derivaten als sehr aufwendig, teuer, oft durch den erforderlichen stöchiometrischen Einsatz (Cer(IV)-Salze, Mangan(III)-sulfat) mit hohen Salzfrachten belastet und meist auch als unspezifisch (R. P. Kreh et al., Org. Chem., 1989, 54, 1526-1531; M. Hudlicky, Oxidations in Organic Chemistry, ACS Monograph 186, Washington/DC, 1990, S. 92-98; T. A. Gorodetskaya et al., U.S.S.R. Patent 1 121 255, 1984; Chem. Abstr., 1985, 102, 203754; W. Adam et al., Synthesis, 1993, 280-282, J. Skarzewski, Tetrahedron, 1984, 40, 4997-5000; S. Yamaguchi et al., Bull. Chem. Soc. Jpn., 1986, 59, 2881-2884; M. Periasamy, M. V. Bhatt, Tetrahedron Lett. 1978, 4561-4562; Y. Asakawa et al., 1988, J. Org. Chem., 53, 5453-5457; W. Chen, Chem. Abstr, 1987, 107, 58620).

Arbeiten von Buchler et al. (DE-A-3731689, DE-A-3731690) haben gezeigt, daß Rhenium-Komplexe Olefine epoxidieren, nicht aber Aromaten.

Aus der EP-A-380085 sind rheniumorganische Verbindungen bekannt, die als Katalysatoren zur Oxidation von Olefinen in Gegenwart von Wasserstoffperoxid eingesetzt werden. Dabei wurde festgestellt, daß andere Rheniumverbindungen, namentlich $Re_2O_7$, $ReO_3$, $ReO_2$ sowie $(CH_3)_3SnOReO_3$, keinerlei katalytische Aktivitäten bei der versuchten Oxidation von Olefinen entfalten.

Aus der prioritätsälteren, nicht vorveröffentlichten DE-Anmeldung (P 4402333.2) sind Organorheniumoxide bekannt, die effektiv als selektiv wirkende Katalysatoren zur Oxidation einer Vielzahl aromatischer Verbindungen zu chinoiden Systemen, in Gegenwart von peroxidhaltigen Reagenzien eingesetzt werden können.

Nachteilig ist, daß der Katalysator vor seiner Verwendung aus einfacheren Rheniumverbindungen hergestellt werden muß, was einen gewissen Zeitaufwand und zusätzliche Kosten impliziert.

Es besteht daher die Aufgabe, ein möglichst leicht zugängliches, wohlfeiles, einfach handhabbares, lagerfähiges, wirksames Katalysatorsystem zu finden, das die gewünschte Selektivität bei der Oxidation von Aromaten erreicht.

Gegenstand der Erfindung ist die Verwendung von Verbindungen der allgemeinen Formel

$$Re_a\,O_b\text{-}X_c\,L_d \qquad\qquad (I),$$

worin

X = F, Cl, Br, J oder OH,
L = Lewis-Base,
a = eine ganze Zahl 1, 2 oder 3
b = Null oder eine ganze Zahl von 2 bis 9,
c = Null oder eine ganze Zahl von 1 bis 9,
d = Null oder eine ganze Zahl von 1 bis 6,

und die Summe von a, b und c so ist, daß sie der Null- bzw. Drei- bis Siebenwertigkeit des Rheniums gerecht wird mit der Maßgabe, daß, falls b ungleich Null, $b \geq 2 \cdot a$ ist, als Katalysatoren zur Oxidation von elektronenreichen aromatischen Verbindungen sowie deren Derivaten.

Beispiele für die Lewis-Base L sind Pyridin, Bipyridin, t-Butylpyridin, Amine, insbesondere sekundäre und tertiäre Amine wie Triethylamin und Chinuclidin, $H_2O$ und Polyether wie Diglyme.

Bevorzugt verwendet werden Verbindungen, worin b eine ganze Zahl von 2 bis 9, c Null oder 1 ist und X, L, a und d die obengenannte Bedeutung haben.

Besonders bevorzugt verwendet werden die Rheniumoxide $ReO_2$, $ReO_3$ und $Re_2O_7$, Rheniumtrioxofluorid

(FReO$_3$) und Rheniumtrioxochlorid (ClReO$_3$), sowie ihre entsprechenden Lewis-Basen L-Addukte.

Ganz besonders bevorzugt sind ReO$_3$ und Re$_2$O$_7$.

Weiterer Gegenstand der Erfindung ist ein Verfahren zur Oxidation von elektronenreichen aromatischen Verbindungen, das dadurch gekennzeichnet ist, daß elektronenreiche C$_6$-C$_{22}$-Arylverbindungen und deren Derivate in Gegenwart von einem Katalysator der allgemeinen Formel Re$_a$O$_b$X$_c$L$_d$ (I), worin X, L, a, b, c und d die obengenannte Bedeutung haben, und einer peroxidhaltigen Verbindung in einem organischen Lösemittel oxidiert werden.

Geeignete Arylverbindungen für das erfindungsgemäße Verfahren sind elektronenreiche aromatische Verbindungen oder kondensierte aromatische Systeme mit 6 bis 22 C-Atomen, bevorzugt mit 6 bis 14 C-Atomen, die gegebenenfalls ein- oder mehrfach, gleich oder verschieden mit einer Elektronendonatorgruppe substituiert sein können. Typische geeignete Elektronendonatorgruppen sind Hydroxy, C$_1$-C$_3$-Alkoxy, N-Acylamino, N-Acylamino-C$_1$-C$_3$-alkyl, Acyloxyund C$_1$-C$_3$-Alkyl.

Beispiele für solche Arylverbindungen sind Xylole, zwei, drei- oder vierfach substituierte C$_1$-C$_3$-Alkylbenzole oder C$_1$-C$_3$-Alkoxybenzole, Naphthalin und seine ein- bis sechsfach substituierten C$_1$-C$_3$-Alkyl- oder C$_1$-C$_3$-Alkoxyderivate, Anthracen und seine C$_1$-C$_3$-Alkyl- oder C$_1$-C$_3$-Alkoxyderivate, Phenanthren und höher kondensierte Aromaten, Phenol, Hydrochinon, Resorcin, Brenzcatechin und Pyrogallol, aber auch Biphenyl.

Bevorzugte Arylverbindungen sind Naphthalin und Anthracen sowie ihre Derivate, besonders bevorzugt ist Naphthalin und seine Derivate, insbesondere 2-Methylnaphthalin.

Durch das erfindungsgemäße Verfahren werden die Arylverbindungen im allgemeinen zu den entsprechenden chinoiden Systemen oxidiert. Aus 2-Methylnaphthalin erhält man beispielsweise das 2-Methyl-1,4-naphthochinon, den Grundkörper der Vitamin-K-Reihe.

Bei höhersubstituierten (dreifach und mehr) Arylverbindungen bei denen die Ausbildung eines chinoiden Systems nicht möglich ist, wird durch das erfindungsgemäße Verfahren die entsprechende Hydroxylverbindung hergestellt. Typische Beispiele für solche höhersubstituierten Arylverbindungen sind 1,2,3,5,8-Pentamethylnaphthalin; 1,2,3-Trimethylbenzol, Mesitylen und 1,3,5-Trimethoxybenzol. Aus diesen Ausgangsstoffen erhält man beispielsweise nach dem erfindungsgemäßen Verfahren folgende Hydroxylverbindungen: 4-Hydroxy-1,2,3,5,8-pentamethyl-naphthalin, 1-Hydroxy-3,4,5-trimethylbenzol, 1-Hydroxy-2,4,6-trimethylbenzol und 1-Hydroxy-2,4,6-trimethoxybenzol.

Nach dem erfindungsgemäßen Verfahren wird die zu oxidierende aromatische Verbindung in einem organischen Lösemittel gelöst und mit dem Katalysator versetzt. Die Konzentration der gelösten aromatischen Verbindungen beträgt 0,1 mol in 10-1000 ml, bevorzugt 0,1 mol in 25-250 ml, besonders bevorzugt 0,1 mol in 50-200 ml Lösemittel. Geeignete organische Lösemittel sind beispielsweise Eisessig, THF, tert.-Butanol oder tert.-Butylmethylether, vorzugsweise Eisessig oder THF. Der Katalysator kann in einer Menge von 0,01-10,0 Mol-%, vorzugsweise 0,1-2,0 Mol-%, eingesetzt werden. Zu dieser Lösung wird die peroxidhaltige Verbindung (5-90 Gew.-%) in einem molaren Verhältnis von 1:1 bis zu 20:1, in bezug auf die zu oxidierenden aromatischen Verbindungen gegeben.

Die Reaktion ist weitgehend pH-unabhängig; bevorzugt wird die Reaktion bei pH $\leq$ 7 durchgeführt.

Die Reaktionsmischung wird bis zum vollständigen Umsatz bei einer Temperatur von 10-100°C, vorzugsweise 20-70°C, gerührt. Danach wird die Reaktionsmischung in für den Fachmann üblicher Weise aufgearbeitet, d.h. beispielsweise neutralisiert, extrahiert und getrocknet. Das Rohoxidationsprodukt kann beispielsweise durch Hochvakuumdestillation oder durch Umkristallisation weiter gereinigt werden.

Die Rheniumverbindungen der Formel I sind als handelsübliche Verbindungen (ReO$_2$, ReO$_3$, Re$_2$O$_7$) käuflich erhältlich oder leicht herstellbar (G. Brauer, Handbuch der Präparativen Anorganischen Chemie, 3. Auflage, Enke-Verlag, Stuttgart 1981). Ihre Eignung als Oxidationskatalysator von Aromaten ist jedoch neu und war keinesfalls zu erwarten (EP-A-380085). Ihr besonderer Vorteil liegt darin, daß sie im allgemeinen käuflich erhältlich, nicht flüchtig und weitgehend pH-unabhängig sind, und sich auf einfache Weise aus dem Oxidationssystem abtrennen lassen. Die Verbindungen der Formel I sind in Kombination mit peroxidhaltigen Verbindungen wie Wasserstoffperoxid, anorganischen Peroxiden, z.B. Alkaliperoxiden, insbesondere Natriumperoxid, sowie Percarbonsäuren und ihren Salzen wie m-Chlorperbenzoesäure, Peressigsäure und Magnesiummonoperoxophthalat hochaktive Katalysatoren für die erfindungsgemäßen Oxidationen.

Bevorzugt eingesetzt werden die Verbindungen der Formel I in Kombination mit Wasserstoffperoxid, dessen Konzentration im Bereich von 5-90 %, vorzugsweise 60-85 % liegen kann. Handelsübliches Perhydrol (= 30 %iges H$_2$O$_2$) kann auch verwendet werden.

Beispiele

Allgemeine Arbeitsvorschrift zur rheniumkatalysierten Oxidation von aromatischen Verbindungen

Die zu oxidierenden Substrate wurden in Eisessig, THF oder Mischungen davon gelöst und mit dem Katalysator versetzt. Als letztes wurde Wasserstoffperoxid als Oxidationsmittel zugegeben. Die Reaktionsmischung wurde bis zum vollständigen Umsatz bei 20, 40 oder 70°C (s. Tabelle) gerührt.

Aufarbeitung:

Die Reaktionslösung wurde mit einer gesättigten Natriumhydrogencarbonat-Lösung neutralisiert. Die wäßrige Mutterlauge wurde dreimal mit Methylenchlorid extrahiert, die vereinigten Extrakte wurden über $MgSO_4$ getrocknet. Das Lösemittel wurde dann im Vakuum entfernt. Nach Entfernung des Methylenchlorides wurden im allgemeinen gelb gefärbte, feste Oxidationsprodukte erhalten. Die gemäß obiger Arbeitsvorschrift durchgeführten Beispiele sind Tabelle 1 zu entnehmen.

Tabelle 1: Oxidationsbeispiele nach der allgemeinen Arbeitsvorschrift mit zugehörigen Reaktionsbedingungen

| Nr. | Arylverbindung (jeweils 10 mmol) | Katalysator (jeweils 0,2 mmol) | t[h] | T [°C] | Lösemittel | Ausbeute (Gew.-%) | Produkt |
|---|---|---|---|---|---|---|---|
| 1 | 2,3-Dimethylnaphthalin | $ReO_3$[γ] | 4 | 20 | AcOH/THF[a] | 30 | 2,3-Dimethylnaphthochinon |
| 2 | 2,3-Dimethylnaphthalin | $Re_2O_7$ · Bipyridin[α] | 4 | 20 | AcOH/THF[a] | 43 | 2,3-Dimethylnaphthochinon |
| 3 | 2,3-Dimethylnaphthalin | $FReO_3$ · Bipyridin[α] | 4 | 20 | AcOH/THF[a] | 11 | 2,3-Dimethylnaphthochinon |
| 4 | 2,3-Dimethylnaphthalin | $ClReO_3$ · Bipyridin[α] | 4 | 20 | AcOH/THF[a] | 74 | 2,3-Dimethylnaphthochinon |
| 5 | 2,3-Dimethylnaphthalin | $BrReO_3$ · Bipyridin[α] | 4 | 20 | AcOH/THF[a] | 5 | 2,3-Dimethylnaphthochinon |
| 6 | 2,3-Dimethylnaphthalin | $C_6H_{14}N_3ReO_3ReO_4$[α] | 4 | 20 | AcOH/THF[a] | 11 | 2,3-Dimethylnaphthochinon |
| 7 | 2,3-Dimethylnaphthalin | $ReO_3$[α] | 24 | 20 | THF[b] | 43 | 2,3-Dimethylnaphthochinon |
| 8 | 2,3-Dimethylnaphthalin | $Re_2O_7$[α] | 24 | 20 | THF[b] | 51 | 2,3-Dimethylnaphthochinon |
| 9 | 2,3-Dimethylnaphthalin | $ClReO_3$[α] | 4 | 20 | THF[b] | 23 | 2,3-Dimethylnaphthochinon |
| 10 | 2,3-Dimethylnaphthalin | $ClReO_3$ · Bipyridin[α] | 5 | 20 | THF[b] | 36 | 2,3-Dimethylnaphthochinon |
| 11 | Mesitylen | $ReO_3$[α] | 4 | 20 | AcOH[c] | 69 | 4-Hydroxymesitylen |

$C_6H_{14}N_3$ = 1,3,7-Triazacyclononan

Solvens:    a) 22 ml AcOH + THF (THF-Menge ausreichend zum vollständigen Lösen des Substrates nach $H_2O_2$-Zugabe)
          b) 22 ml THF
          c) 22 ml AcOH ( = Eisessig);

85 % $H_2O_2$; Molverhältnis Arylverbindung/$H_2O_2$ = α) 1:20, ß) 1:15, γ) 1:10, δ) 1:7, ε) 1:5, η) 1:3, θ) 1:1

EP 0 686 618 B1

| Nr. | Arylverbindung (jeweils 10 mmol) | Katalysator (jeweils 0,2 mmol) | t[h] | T [°C] | Lösemittel | Ausbeute (Gew.-%) | Produkt |
|---|---|---|---|---|---|---|---|
| 12 | Mesitylen | $Re_2O_7$ [α)] | 4 | 20 | AcOH [c)] | 14 / 37 | 4-Hydroxymesitylen / 1,3-Dihydroxymesitylen |
| 13 | Mesitylen | $Re_2O_7$ [γ)] | 2 | 40 | AcOH [c)] | 73 | 4-Hydroxymesitylen |
| 14 | Mesitylen | $Re_2O_7$ [ε)] | 1 | 20 | AcOH [c)] | 38 | 4-Hydroxymesitylen |
| 15 | 1,2,3-Trimethylbenzol | $Re_2O_7$ [β)] | 0,17 | 70 | AcOH [c)] | 34 | 3,4,5-Trimethylphenol |
| 16 | 1,3,5-Trimethoxybenzol | $Re_2O_7$ [θ)] | 1 | 20 | AcOH [c)] | 20 | 2,4,6-Trimethoxyphenol |
| 17 | 2-Methylnaphthalin | $Re_2O_7$ [δ)] | 4 | 20 | AcOH [c)] | 57 | 2-Methylnaphthochinon |
| 18 | 2-Methylnaphthalin | $Re_2O_7$ [η)] | 4 | 20 | AcOH [c)] | 33 | 2-Methylnaphthochinon |
| 19 | 2-Methylnaphthalin | $Re_2O_7$ [η)] | 4 | 40 | AcOH [c)] | 57 | 2-Methylnaphthochinon |
| 20 | 2-Methylnaphthalin | $Re_2O_7$ [δ)] | 4 | 40 | AcOH [c)] | 70 | 2-Methylnaphthochinon |

**Patentansprüche**

1. Verwendung von Verbindungen der allgemeinen Formel

$$Re_a\,O_b\,X_c\,L_d \qquad\qquad\text{(I),}$$

worin

X = F, Cl, Br, J oder OH,
L = Lewis-Base,
a = eine ganze Zahl 1, 2 oder 3
b = Null oder eine ganze Zahl von 2 bis 9,
c = Null oder eine ganze Zahl von 1 bis 9,
d = Null oder eine ganze Zahl von 1 bis 6,

und die Summe von a, b und c so ist, daß sie der Null- bzw. Drei- bis Siebenwertigkeit des Rheniums gerecht wird mit der Maßgabe, daß, falls b ungleich Null, b $\geq$ 2 $\cdot$ a ist, als Katalysatoren zur Oxidation von elektronenreichen aromatischen Verbindungen sowie deren Derivaten.

2. Verwendung von Verbindungen der Formel I nach Anspruch 1, dadurch gekennzeichnet, daß b eine ganze Zahl von 2 bis 7 und c Null oder 1 ist.

3. Verfahren zur Oxidation von elektronenreichen aromatischen Verbindungen, dadurch gekennzeichnet, daß elektronenreiche $C_6$-$C_{22}$-Arylverbindungen und deren Derivate in Gegenwart von einem Katalysator der allgemeinen Formel $Re_aO_bX_cL_d$(I), worin

X = F, Br, Cl, J oder OH,
L = Lewis Base,
a = eine ganze Zahl 1, 2 oder 3,
b = Null oder eine ganze Zahl von 2 bis 7,
c = Null oder eine ganze Zahl von 1 bis 9,
d = Null oder eine ganze Zahl von 1 bis 6,

und die Summe von a, b und c so ist, daß sie der Null- bzw. Drei- bis Siebenwertigkeit des Rheniums gerecht wird mit der Maßgabe, daß, falls b ungleich Null, b $\geq$ 2 $\cdot$ a ist, und einer peroxidhaltigen Verbindung in einem organischen Lösemittel oxidiert werden.

4. Verfahren gemäß Anspruch 3, dadurch gekennzeichnet, daß als peroxidhaltige Verbindung Wasserstoffperoxid eingesetzt wird.

5. Verfahren gemäß Anspruch 3 oder 4, dadurch gekennzeichnet, daß eine $C_6$-$C_{14}$-Arylverbindung oxidiert wird.

6. Verfahren gemäß einem oder mehreren der Ansprüche 3 bis 5, dadurch gekennzeichnet, daß Naphthalin und seine Derivate oxidiert werden.

7. Verfahren gemäß einem oder mehreren der Ansprüche 3 bis 6, dadurch gekennzeichnet, daß 2-Methylnaphthalin oxidiert wird.

**Claims**

1. The use of compounds of the formula

$$Re_aO_bX_cL_d \qquad\qquad\text{(I)}$$

in which

X = F, Cl, Br, I or OH,
L = Lewis base,

a = an integer 1, 2 or 3
b = zero or an integer from 2 to 9,
c = zero or an integer from 1 to 9,
d = zero or an integer from 1 to 6,

and the sum of a, b and c is such that it is appropriate to the zerovalency or trivalency to heptovalency of rhenium with the proviso that if b is not equal to zero, $b \geq 2 \cdot a$, as catalysts for the oxidation of electron-rich aromatic compounds and derivatives thereof.

2. The use of compounds of the formula I as claimed in claim 1, wherein b is an integer from 2 to 7 and c is zero or 1.

3. A process for the oxidation of electron-rich aromatic compounds, which comprises oxidizing in an organic solvent electron-rich $C_6$-$C_{22}$-aryl compounds and derivatives thereof in the presence of a catalyst of the formula $Re_aO_bX_cL_d$ (I), in which

X = F, Cl, Br, I or OH,
L = Lewis base,
a = an integer 1, 2 or 3
b = zero or an integer from 2 to 9,
c = zero or an integer from 1 to 9,
d = zero or an integer from 1 to 6,

and the sum of a, b and c is such that it is appropriate to the zerovalency or trivalency to heptovalency of rhenium with the proviso that if b is not equal to zero, $b \geq 2 \cdot a$, and of a peroxide-containing compound.

4. The process as claimed in claim 3, wherein the peroxide-containing compound used is hydrogen peroxide.

5. The process as claimed in claim 3 or 4, wherein a $C_6$-$C_{14}$-aryl compound is oxidized.

6. The process as claimed in one or more of claims 3 to 5, wherein naphthalene and derivatives thereof are oxidized.

7. The process as claimed in one or more of claims 3 to 6, wherein 2-methylnaphthalene is oxidized.

**Revendications**

1. Utilisation de composés de formule générale (I)

$$Re_a \, O_b \, X_c \, L_d \qquad\qquad (I),$$

où

X = F, Cl, Br, J ou OH,
L = base de Lewis,
a = un entier de valeur 1, 2 ou 3,
b = 0 ou un entier de 2 à 9,
c = 0 ou un entier de 1 à 9,
d = 0 ou un entier de 1 à 6,

et la somme de a, b et c est telle qu'elle soit équitable à la valence 0, respectivement à la tri- à l'heptavalence du rhénium, à la condition que si b est différent de 0, $b \geq 2.a$, comme catalyseurs pour l'oxydation de composés aromatiques riches en électrons, de même que leur dérivés.

2. Utilisation de composés de formule I selon la revendication 1, caractérisée en ce que b est un entier de 2 à 7 et c vaut zéro ou 1.

3. Procédé pour l'oxydation de composés aromatiques riches en électrons, caractérisé en ce que l'on oxyde des composés aryles en $C_6$-$C_{22}$ riches en électrons et leurs dérivés en présence d'un catalyseur de formule générale $Re_aO_bX_cL_d$ (I), où

X = F, Cl, Br, J ou OH,
L = base de Lewis,
a = un entier de valeur 1, 2 ou 3,
b = 0 ou un entier de 2 à 9,
c = 0 ou un entier de 1 à 9,
d = 0 ou un entier de 1 à 6,

et la somme de a, b et c est telle qu'elle soit équitable à la valence 0, respectivement à la tri- à l'heptavalence du rhénium, a la condition que si b est différent de 0, b≥2.a , et on oxyde avec un composé peroxydique dans un solvant organique.

4. Procédé selon la revendication 3, caractérisé en ce que l'on emploie comme composés peroxydiques du peroxyde d'hydrogène.

5. Procédé selon la revendication 3 ou 4, caractérisé en ce qu'on oxyde un composé aryle en $C_6$-$C_{14}$.

6. Procédé selon une ou plusieurs des revendications de 3 à 5, caractérisé en ce que l'on oxyde le naphtalène et ses dérivés.

7. Procédé selon une ou plusieurs des revendications 3 à 6, caractérisé en ce qu'on oxyde le 2-méthylnaphtalène.